# EUROPEAN PATENT SPECIFICATION

(11) **EP 4 006 170 B1**
(45) Date of publication and mention of the grant of the patent: **11.02.2026**
(21) Application number: 20843278.1
(22) Date of filing: 24.07.2020
(51) Int. Cl.: C12Q 1/6823, C12Q 1/6876, C12Q 1/6844, C12N 9/22, C12N 15/11

(54) **CRISPR MULTI-TARGET DETECTION METHOD AND TEST KIT THEREFOR**
VERFAHREN ZUR CRISPR-MULTITARGET-DETEKTION UND TESTKIT DAFÜR
PROCÉDÉ DE DÉTECTION MULTI-CIBLE CRISPR ET KIT DE TEST ASSOCIÉ

(30) Priority: 24.07.2019 CN 201910673246
(43) Date of publication of application: 01.06.2022
(73) Proprietor: Shanghai Tolo Biotechnology Company Limited, Shanghai 200032 (CN)
(72) Inventor: WANG, Jin, Shanghai 200032 (CN); LI, Shiyuan, Shanghai 200032 (CN)
(74) Representative: Ipsilon
(86) International application number: PCT/CN2020/104588
(87) International publication number: WO 2021/013257

(56) References cited:
- WO-A1-2017/147345
- WO-A1-2019/011022
- CA-A1- 3 069 788
- CN-A- 109 312 336
- US-A1- 2014 212 869
- US-A1- 2015 211 058
- ALEXANDRA EAST-SELETSKY ET AL: "Two distinct RNase activities of CRISPR-C2c2 enable guide-RNA processing and RNA detection", NATURE, vol. 538, no. 7624, 26 September 2016 (2016-09-26), London, pages 270 - 273, XP055719305, ISSN: 0028-0836, DOI: 10.1038/nature19802
- SHI-YUAN LI ET AL: "CRISPR-Cas12a-assisted nucleic acid detection", CELL DISCOVERY, vol. 4, no. 1, 24 April 2018 (2018-04-24), XP055538799, DOI: 10.1038/s41421-018-0028-z
- JANICE S. CHEN ET AL: "CRISPR-Cas12a target binding unleashes indiscriminate single-stranded DNase activity", SCIENCE, vol. 360, no. 6387, 27 April 2018 (2018-04-27), US, pages 436 - 439, XP055615609, ISSN: 0036-8075, DOI: 10.1126/science.aar6245
- ALEXANDRA EAST-SELETSKY; MITCHELL R O’CONNELL; SPENCER C KNIGHT; DAVID BURSTEIN; JAMIE H D CATE; ROBERT TJIAN; JENNIFER A DOUDNA: "Two Distinct RNase Activities of CRISPR-C2c2 Enable Guide RNA Processing and RNA Detection", NATURE, vol. 538, 7624, 13 October 2016 (2016-10-13), pages 270 - 273, XP055719305, ISSN: 0028-0836, DOI: 10.1038/nature19802
- FANG, RUI ET AL: "New Method of Genome Editing Derived From CRISPR/Cas9", PROGRESS IN BIOCHEMISTRY AND BIOPHYSICS, vol. 40, no. 8, 31 August 2013 (2013-08-31), pages 691 - 702, XP055320441, ISSN: 0496-3490, DOI: 10.3724/SP.J.1206.2013.00215
- LI JUN, ZHANG YI, CHEN KUN-LING, SHAN QI-WEI, WANG YAN-PENG, LIANG ZHEN, GAO CAI-XIA: "CRISPR/Cas: a novel way of RNA-guided genome editing", HEREDITAS, vol. 35, no. 11, 14 October 2013 (2013-10-14), pages 1265 - 1273, XP055775588, ISSN: 0253-9772, DOI: 10.3724/SP.J.1005.2013.01265
- JONATHAN S GOOTENBERG; OMAR O ABUDAYYEH; JEONG WOOK LEE; PATRICK ESSLETZBICHLER; AARON J DY; JULIA JOUNG; VANESSA VERDINE; NINA DO: "Nucleic acid detection with CRISPR-Cas13a/C2c2", SCIENCE, vol. 356, no. 2336, 28 April 2017 (2017-04-28), pages 438 - 442, XP055481345, ISSN: 0036-8075, DOI: 10.1126/science.aam9321

## Description

### TECHNICAL FIELD

The present invention relates to the field of biotechnology, in particular, the present invention relates to a CRISPR multi-target detection method and a kit therefor.

### BACKGROUND

CRISPR diagnostic method using Cas13 or Cas12 protein is known as the next generation detection technology (called SHERLOCK and HOLMES respectively), because it is fast, sensitive, specific, simple and cheap. Cas12 and Cas13 proteins can be used for nucleic acid detection based on their viability to collateral (or trans) cleavage, that is, under the guidance of artificially designed guide RNA, they combine with nucleic acid fragments of specific sequences and then cut single-stranded nucleic acid probes, thereby generating a detectable signal. The difference between Cas12 and Cas13 is that the Cas13 protein binds the RNA target and cleaves the RNA probe, while Cas12 binds the DNA target and cleaves the single-stranded DNA probe.

In principle, if Cas13 is used to detect conventional DNA targets, the target DNA is amplified, and promoter sequences such as T7 are introduced in the process. Then *in vitro* transcription is required to generate template RNA for Cas13 recognition and binding. In addition, the RNA reporter probe used in the detection process of Cas13 has a greater risk of being degraded by RNA enzymes, resulting in a higher background signal value of the detection system. In contrast, the Cas12-based detection method is more advantageous because it requires neither *in vitro* transcription nor RNA reporter probes.

The methods and techniques of simultaneously detecting multiple targets in one detection system are very important for the expansion of *in vitro* diagnosis applications. At present, a multi-target detection method based on Cas13 protein has been developed, the key of which is to use the characteristics of different species-derived Cas13 proteins for different RNA reporter probes with different collateral cleavage activities. However, there is no research report on this feature of Cas12. Therefore, it is necessary to develop a new method to use Cas protein for multi-target nucleic acid detection.

### SUMMARY OF THE INVENTION

The object of the present invention is to provide a CRISPR-Cas protein-based multi-target detection method and a kit. The invention is defined by the appended claims.

In the first aspect of the present invention, it provides a detection system for detecting target nucleic acid molecules, which comprises:
(a) n kinds of guide RNA-reporter nucleic acid complex probes, which has a structure as shown in Formula Ia, Ib, Ic or Id,

   Z1-Z2-Z3-Z4-Z5 (Formula Ia)

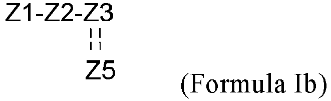

   Z5-Z4-Z3-Z2-Z1 (Formula Ic)

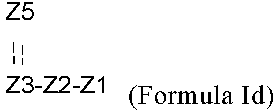

   wherein,
   Z1 is a first stem-loop structure region;
   Z2 is none or a nucleic acid linker region;
   Z3 is a guide RNA region, wherein the Z3 comprises a nucleic acid sequence that can guide the Cas protein to specifically bind to a target nucleic acid molecule;
   Z5 is a single-stranded nucleic acid to be cleaved with a detectable label, wherein the detectable label presents a different detection state when the single-stranded nucleic acid to be cleaved is cleaved and not cleaved, thereby being detected;
   **wherein,** when the target nucleic acid does not exist in the detection system, Z3 and Z5 form a complementary paired double-stranded structure region, and when the target nucleic acid exists in the detection system, Z3 and Z5 do not form the complementary paired double-stranded structure region;
   Z4 is none, or a chemical bond or a linker region used for connecting Z3 and Z5;
   " 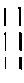 " is a hydrogen bond for complementary base pairing;
   and, n is a positive integer with n ≥ 1; and
(b) Cas protein, which is a Cas protein with collateral single-stranded nucleic acid cleavage activity.

In another preferred embodiment, the Cas protein is selected from the group consisting of Cas12 type, Cas13a type, Cas13b type, Cas14 type, CasΦ, and a combination thereof.

In another preferred embodiment, when the Cas protein is Cas12 type and/or Cas13a type and/or Cas14 type, the guide RNA-reporter nucleic acid complex probe has a structure as shown in Formula Ia or Ib.

In another preferred embodiment, when the Cas protein is of type Cas13b, the guide RNA-reporter nucleic acid complex probe has a structure as shown in Formula Ic or Formula Id.

In another preferred embodiment, the Cas12 type is selected from the group consisting of Cas12a, Cas12b, Cas12d, Cas12g, Cas12i, and a combination thereof.

In another preferred embodiment, when the target nucleic acid does not exist in the detection system, the guide RNA-report nucleic acid complex probe is of Formula IIa structure: 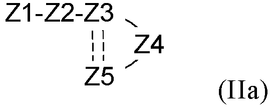 wherein,
Z1, Z2, Z3, Z4 and Z5 are as described above,
" 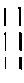 " is a hydrogen bond for complementary base pairing.

In another preferred embodiment, the "Z1-Z2-Z3" in Formula Ia, Formula IIa, Formula Ic, Formula Ib, and the "Z3-Z2-Z1" in Formula Id are in a direction from 5' to 3'.

In another preferred embodiment, when the target nucleic acid does not exist in the detection system, the guide RNA-report nucleic acid complex probe is of Formula IIc structure: 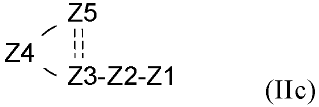 wherein,
Z1, Z2, Z3, Z4 and Z5 are as described above,
" 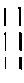 " is a hydrogen bond for complementary base pairing.

In another preferred embodiment, the complementary paired double-stranded structure region comprises a double-stranded structure region formed by partial or total complementary pairing of Z3 and Z5.

In another preferred embodiment, the complementary paired double-stranded structure region is a double-stranded structure region formed by total complementary pairing of Z3 and Z5.

In another preferred embodiment, the Z1, Z2 and Z3 are used to guide the binding of the Cas protein to the target nucleic acid.

In another preferred embodiment, the Z1 is used to bind or anchor the Cas protein.

In another preferred embodiment, the guide RNA region guides the Cas protein to bind to the target nucleic acid by complementary pairing with the target nucleic acid.

In another preferred embodiment, Z3, Z4 and Z5 form a second stem-loop structure region, wherein Z4 is a loop region (including simple structure and complex structure loop region).

In another preferred embodiment, the Z1 is substantially or entirely composed of RNA.

In another preferred embodiment, the Z3 is substantially or entirely composed of RNA.

In another preferred embodiment, the guide RNA-reporter nucleic acid complex probe is single-stranded.

In another preferred embodiment, the stem-loop structure in Z1 is a crRNA (or a CRISPR RNA) stem-loop structure.

In another preferred embodiment, the length of the Z1 is 10-300nt, preferably 19-100nt, more preferably 19-91nt.

In another preferred embodiment, the Z2 is none or a nucleic acid linker region with a length of 0-20nt.

In another preferred embodiment, in the system:
(i) n ≥ 2, and n is a positive integer;
(ii) the detectable label is a fluorescent group, and Z5 has a fluorescent group, while Z4 and/or Z5 also has a quenching group, and the fluorescent signal emitted by the fluorescent group can be detected when and only when the single-stranded nucleic acid to be cleaved is cleaved; and/or
(iii) among the n kinds of guide RNA-reporter nucleic acid complex probes, the fluorescent groups are different from each other, so that they can be distinguished.

In another preferred embodiment, the detectable label is a fluorescent group, and Z5 has a quenching group, while Z4 and/or Z5 also has a fluorescent group, and the fluorescent signal emitted by the fluorescent group can be detected when and only when the single-stranded nucleic acid to be cleaved is cleaved.

In another preferred embodiment, the detectable label is a fluorescent group, wherein the fluorescent group is located in any one of Z5, Z4 and Z3, and the quenching group is located in any segment of Z5, Z4 and Z3, and the fluorescent signal emitted by the fluorescent group can be detected when and only when the single-stranded nucleic acid to be cleaved is cleaved.

In another preferred embodiment, the fluorescent group and the quenching group are not located at Z3 at the same time.

In another preferred embodiment, the Z3 contains a nucleic acid sequence that can guide the Cas protein to specifically bind to a target nucleic acid molecule.

In another preferred embodiment, the length of the Z3 is 15-50nt, preferably 16-40nt, more preferably 16-34nt.

In another preferred embodiment, the Z1, Z2 and Z3 are all RNA nucleic acid sequences.

In another preferred embodiment, the Z4 is a DNA and/or RNA nucleic acid sequence.

In another preferred embodiment, the Z5 is a DNA single-stranded nucleic acid sequence, or an RNA single-stranded nucleic acid sequence, or a nucleic acid sequence having both RNA and DNA.

In another preferred embodiment, the Z5 contains nucleotides based on natural bases or nucleotides based on natural bases and unnatural bases.

In another preferred embodiment, the nucleotide comprises ribonucleic acid, deoxyribonucleic acid, peptide nucleic acid, and a combination thereof.

In another preferred embodiment, the natural base is selected from the group consisting of A, T, C, G, U, I.

In another preferred embodiment, the length of the Z5 is 3-50nt, preferably 4-30nt, more preferably 6-12nt.

In another preferred embodiment, the labels carried in Z5 are a fluorescent group and a quenching group.

In another preferred embodiment, the fluorescent group and the quenching group are each independently located at the 5' end, 3' end and/or the middle of the Z5.

In another preferred embodiment, in the reaction system, each guide RNA-reporter nucleic acid complex probe has different fluorescent groups and different or the same quenching groups.

In another preferred embodiment, the detection system further comprises m kinds of target nucleic acid molecules to be detected, wherein m is a positive integer, and m ≤ n.

In another preferred embodiment, the target nucleic acid molecules comprise target nucleic acid molecules derived from the group consisting of a plant, an animal, an insect, a microorganism, a virus, and a combination thereof.

In another preferred embodiment, the target nucleic acid is a synthetic or naturally occurring nucleic acid.

In another preferred embodiment, the target nucleic acid comprises a wild-type or mutant nucleic acid.

In another preferred embodiment, the target nucleic acid molecule is a target DNA or a target RNA.

In another preferred embodiment, the target DNA comprises non-reverse-transcribed DNA or DNA obtained by reverse transcription or amplification of RNA (e.g., cDNA, etc.).

In another preferred embodiment, the target RNA comprises an RNA that is not transcribed or obtained by DNA transcription.

In another preferred embodiment, the detection comprises a qualitative detection or a quantitative detection.

In another preferred embodiment, the detection system further comprises (c) buffer.

In another preferred embodiment, the detection system further comprises target nucleic acid molecules to be detected.

In another preferred embodiment, the detection system further comprises reagents for the nucleic acid amplification reaction.

In another preferred example, the detection system further contains:
(d1) polymerase for amplifying target DNA;
(d2) optional reverse transcriptase for reverse transcription;
(d3) optional transcription enzymes for transcription;
(d4) dNTPs for amplification reactions and/or reverse transcription reactions;
(d5) NTPs for transcription reactions.

In another preferred embodiment, the concentration of the target nucleic acid molecule to be detected in the system to be detected is 1 × 10⁻⁹ nM to 1 × 10³ nM; preferably 1 × 10⁻⁸ nM to 1 × 10² nM.

In another preferred embodiment, the concentration of the target nucleic acid molecule to be detected in the detection system is 1 to 1 x 10¹⁵ copies/ml, preferably 1 to 10¹⁰ copies/ml, more preferably 1 to 10⁵ copies/ml.

In another preferred embodiment, the concentration of the target nucleic acid molecule to be detected in the detection system is 1 to 1000 copies/ml, preferably 1 to 100 copies/ml, more preferably 1 to 10 copies/ml.

In another preferred embodiment, in the detection system, the molar ratio of each guide RNA-reporter nucleic acid complex probe to the corresponding target nucleic acid molecule is 1: 1 to 10¹⁴: 1, preferably 10: 1 to 10⁵: 1, more preferably 20: 1 to 10³: 1.

In another preferred embodiment, the Cas protein is selected from the group consisting of Cas12a, Cas12b, Cas12d, Cas12g, Cas12i, Cas13a, Cas13b, Cas14, and CasΦ.

In another preferred embodiment, the Cas12a protein is selected from the group consisting of FnCas12a, AsCas12a, LbCas12a, Lb5Cas12a, HkCas12a, OsCas12a, TsCas12a, BbCas12a, BoCas12a and Lb4Cas12a.

In another preferred embodiment, the Cas12a protein is LbCas12a or FnCas12a.

In another preferred embodiment, the Cas12b protein is selected from the group consisting of AaCas12b, AacCas12b, AapCas12b, AbCas12b, AkCas12b, AmCas12b, BhCas12b, BsCas12b, EbCas12b and LsCas12b.

In another preferred embodiment, the Cas12g protein is Cas12g1.

In another preferred embodiment, the Cas12i protein is Cas12i1 or Cas12i2.

In another preferred embodiment, the Cas13a protein is selected from the group consisting of LshCas13a, LwaCas13a, LbaCas13a, LseCas13a, LbmCas13a, LbnCas13a, CamCas13a, CgaCas13a, Cga2Cas13a, PprCas13a, LweCas13a, Lwa2Cas13a, LbfCas13a, RcsCas13a, RcrCas13a, RcdCas13a and LbuCas13a.

In another preferred embodiment, the Cas13b protein is selected from the group consisting of BzoCas13b, PinCas13b, PbuCas13b, AspCas13b, PsmCas13b, RanCas13b, PauCas13b, PsaCas13b, Pin2Cas13b, CcaCas13b, PguCas13b, PspCas13b, PigCas13b and Pin3Cas13b.

In another preferred embodiment, the Cas14 protein is selected from the group consisting of Cas14a, Cas14b, Cas14c, Cas14d, Cas14e, Cas14f, Cas14g, Cas14h and Cas14u.

In another preferred embodiment, the CasΦ protein is selected from the group consisting of CasΦ-1, CasΦ-2 and CasΦ-3.

In another preferred embodiment, n is a positive integer between 2 and 200; preferably, n is a positive integer between 2 and 100; more preferably, n is a positive integer between 2 and 20; and more preferably, n is a positive integer between 2 and 10.

In the second aspect of the present invention, it provides a kit for detecting a target nucleic acid molecule, which comprises:
i) a first container and the n kinds of guide RNA-reporter nucleic acid complex probes with the structures as shown in Formula Ia, Ib, Ic or Id located in the first container,

   Z1-Z2-Z3-Z4-Z5 (Formula Ia)

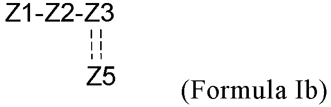

   Z5-Z4-Z3-Z2-Z1 (Formula Ic)

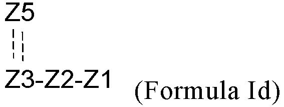

   wherein,
   Z1 is a first stem-loop structure region;
   Z2 is none or a nucleic acid linker region;
   Z3 is a guide RNA region, wherein the Z3 comprises a nucleic acid sequence that can guide the Cas protein to specifically bind to a target nucleic acid molecule;
   Z5 is a single-stranded nucleic acid to be cleaved with a detectable label, wherein the detectable label presents a different detection state when the single-stranded nucleic acid to be cleaved is cleaved and not cleaved, thereby being detected;
   wherein, when the target nucleic acid does not exist in the detection system, Z3 and Z5 form a complementary paired double-stranded structure region, and when the target nucleic acid exists in the detection system, Z3 and Z5 do not form the complementary paired double-stranded structure region;
   Z4 is none, or a chemical bond or a linker region used for connecting Z3 and Z5;
   " 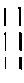 " is a hydrogen bond for c complementary base pairing;
   and, n is a positive integer with n ≥ 1;
ii) a second container and the Cas protein located in the second container, which is a Cas protein with collateral single-stranded nucleic acid cleavage activity;
iii) an optional third container and buffer located in the third container.

In another preferred embodiment, the target nucleic acid molecule is a target DNA and/or a target RNA.

In another preferred embodiment, the first container, the second container and the third container may be the same container or different containers.

In another preferred example, the kit further comprises:
iv) a fourth container and the polymerase for amplifying the target DNA located in the fourth container;
v) an optional fifth container and the reverse transcriptase for reverse transcription and/or the transcriptase for transcription located in the fifth container;
vii) a sixth container and the dNTPs for amplification reactions and/or reverse transcription reactions and/or NTPs for transcription reactions located in the sixth container.

In another preferred embodiment, the detection is used to simultaneously detect two or more different target nucleic acid molecules.

In another preferred embodiment, the detection system further comprises reagents for the nucleic acid amplification reaction.

In another preferred embodiment, the fourth container, the fifth container and the sixth container may be the same container or different containers.

In another preferred embodiment, two, more or all of the first to sixth containers may be the same container or different containers.

In the third aspect of the present invention, it provides a method for detecting a target nucleic acid molecule in a sample, including the following steps:
(i) providing the detection system for simultaneously detecting multiple target nucleic acid molecules as described in the first aspect of the present invention, and the detection system further contains a sample to be detected; and
(ii) detecting whether the guide RNA-reporter nucleic acid complex probe in the detection system is cleaved by the Cas protein, and the cleavage is a collateral (or trans) cleavage for the single-stranded nucleic acid;
wherein, if the guide RNA-reporter nucleic acid complex probe is cleaved by Cas protein, it means that there is a corresponding target nucleic acid molecule in the sample; and the guide RNA-reporter nucleic acid complex probe is not cleaved by Cas protein, it means that there is no corresponding target nucleic acid molecule in the sample.

In another preferred embodiment, the sample to be detected comprises an unamplified sample and an amplified (or nucleic acid amplified) sample.

In another preferred embodiment, the sample to be detected is a sample obtained by amplification.

In another preferred embodiment, the method of nucleic acid amplification is selected from the group consisting of PCR amplification, LAMP amplification, RPA amplification, ligase chain reaction, branched DNA amplification, NASBA, SDA, transcription-mediated amplification, rolling circle amplification, HDA, SPIA, NEAR, TMA and SMAP2.

In another preferred embodiment, the PCR comprises a high temperature PCR, a normal temperature PCR, or a low temperature PCR.

In another preferred embodiment, the detection in step (ii) comprises a fluorescence detection assay.

In another preferred embodiment, the fluorescence detection assay is performed by using a microplate reader, or a fluorescence spectrophotometer or a fluorescence quantitative PCR instrument.

In another preferred embodiment, the method is an *in vitro* detection method.

In another preferred embodiment, the sample is an *in vitro* or *ex vivo* sample.

In another preferred embodiment, the method is non-diagnostic and non-therapeutic.

In another preferred embodiment, the method is diagnostic.

### DESCRIPTION OF DRAWINGS

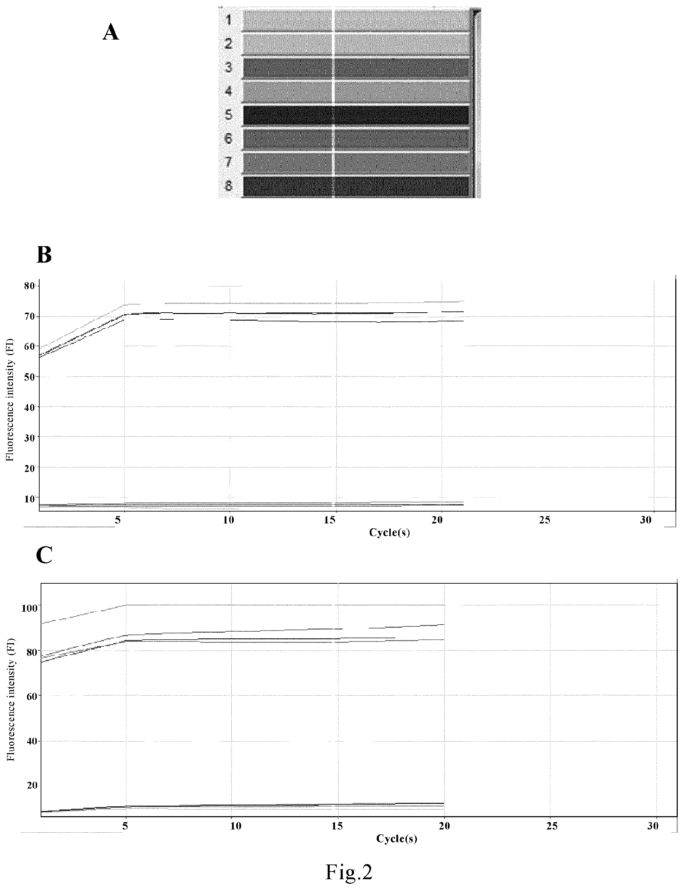

Figure **1** shows a schematic diagram of the guide RNA-reporter nucleic acid complex probe (Formula Ia). In the figure, F represents a fluorescent group (or other detectable label), and Q is a quenching group (or other quenching functional groups used to quench the F signal).
Figure 2 shows the CRISPR multi-target detection results.
Among them, Figure 2A shows the colors representing 8 different samples; Figure 2B is the Green channel: for detecting FAM fluorescence, and the target sequence detected is the DNMT1-3 site; Figure 2C is the Orange channel: for detecting ROX fluorescence, and the target sequence detected is the sry site).

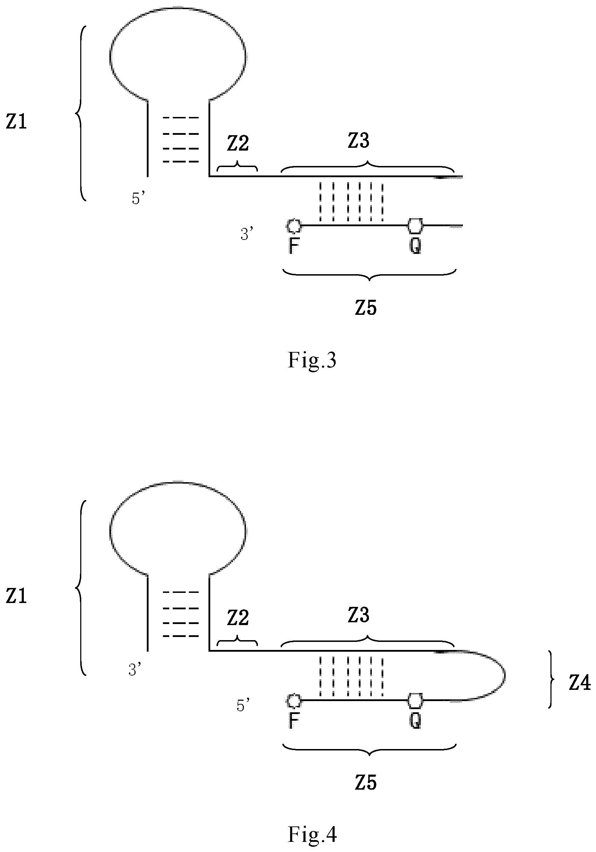

Figure 3 shows a schematic diagram of another guide RNA-reporter nucleic acid complex probe (Formula Ib). In the figure, F represents a fluorescent group (or other detectable labels), and Q is a quenching group (or other quenching functional groups used to quench the F signal).
Figure 4 shows a schematic diagram of another guide RNA-reporter nucleic acid complex probe (Formula Ic). In the figure, F represents a fluorescent group (or other detectable labels), and Q is a quenching group (or other quenching functional groups used to quench the F signal).

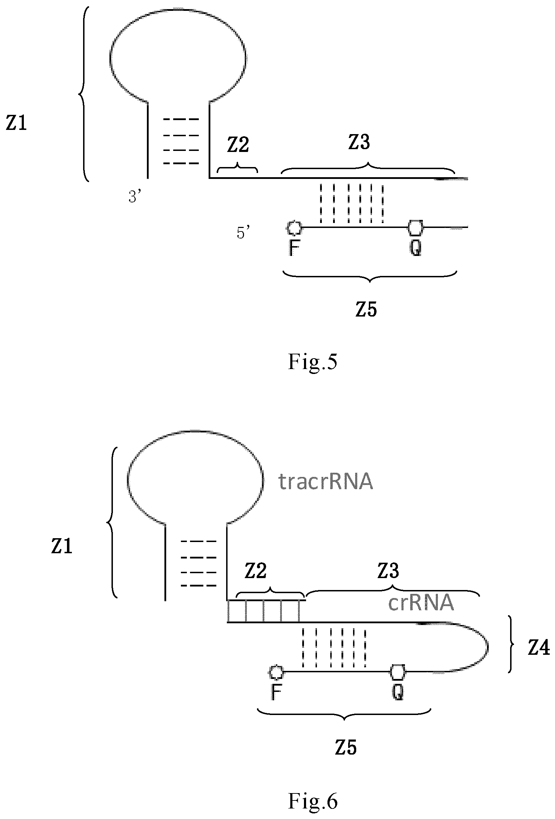

Figure 5 shows a schematic diagram of another guide RNA-reporter nucleic acid complex probe (Formula Id). In the figure, F represents a fluorescent group (or other detectable labels), and Q is a quenching group (or other quenching functional groups used to quench the F signal).
Figure 6 shows the structure of the guide RNA-reporter nucleic acid complex probe in Example 2 or Example 3.

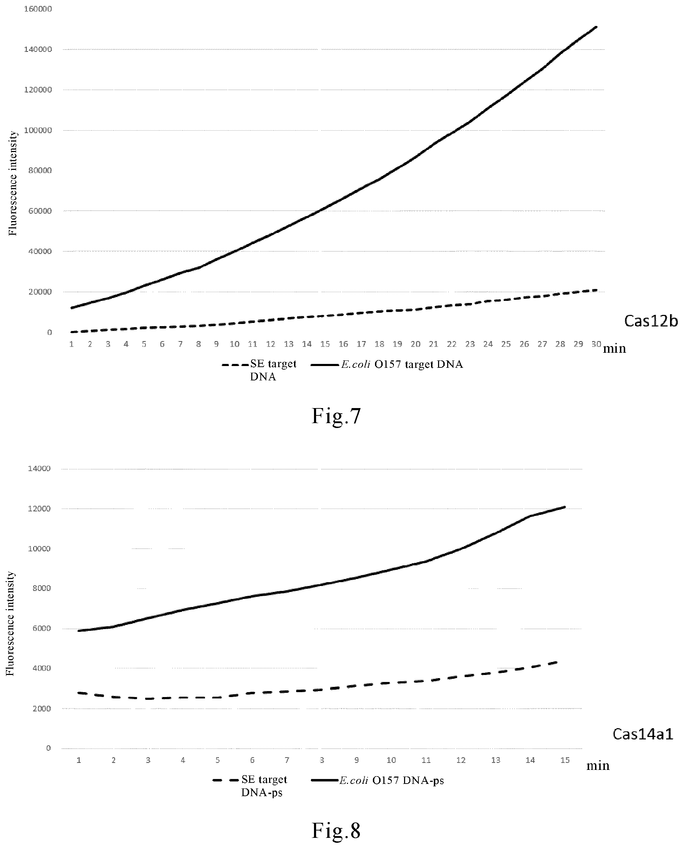

Figure 7 shows the results of the Cas12b multi-target detection test.
Figure 8 shows the results of the Cas14a1 multi-target detection test.

### Detailed Description

After extensive and in-depth research and a large number of screening, the inventors have developed for the first time a method based on CRISPR technology that can simultaneously detect multiple target nucleic acid molecules in the same detection system. Specifically, the present inventors have developed a guide RNA-reporter nucleic acid complex probe, in which a first stem-loop structure region of RNA, a guide RNA region, a linker region, and a single-stranded nucleic acid to be cleaved with a fluorescent group and a quenching group are serially connected. In the same detection system, a variety of different guide RNA-reporter nucleic acid complex probes may be contained, and the fluorescent groups and quenching groups carried by each probe for different target nucleic acid molecules are also different. Therefore, different detected target nucleic acid molecules can be distinguished according to different fluorescence signals detected. Different target nucleic acid molecules contained in the same sample system can be detected simultaneously, quickly and accurately by using this multi-target detection method based on CRISPR technology. The present invention has been completed on this basis.

### Term

The term "CRISPR" refers to clustered regularly interspaced short palindromic repeats, which are part of the immune systems in many prokaryotes.

The term "Cas protein" refers to a CRISPR-associated protein, which is a related protein in the CRISPR system.

The term "Cas12a" (formerly "Cpf1") refers to a crRNA-dependent endonuclease, which is an enzyme of the V-A type in the CRISPR system classification.

The terms "Cas12b", "C2c1" are used interchangeably and refer to a sgRNA-dependent endonuclease, which is an enzyme of the V-B type in the CRISPR system classification.

The terms "Cas12c", "C2c3" are used interchangeably and refer to a tracrRNA:crRNA (or sgRNA)-dependent endonuclease, which is an enzyme of the V-C type in the CRISPR system classification.

The terms "Cas12d", "CasY" are used interchangeably, and refer to a scoutRNA:crRNA-dependent endonuclease, which is an enzyme of the V-D type in the CRISPR system classification.

The term "Cas12g" refers to a tracrRNA:crRNA (or sgRNA)-dependent RNA enzyme, which is an enzyme of the V-G type in the CRISPR system classification.

The term "Cas12i" refers to a crRNA-dependent endonuclease, which is an enzyme of the V-I type in the CRISPR system classification.

The terms "Cas13a", "C2c2" are used interchangeably and refer to a crRNA-dependent endonuclease, which is an enzyme of the VI-A type in the CRISPR system classification.

The term "Cas13b" refers to a crRNA-dependent endonuclease, which is an enzyme of the VI-B type in the CRISPR system classification.

The term "Cas14" refers to a tracrRNA: crRNA (or sgRNA)-dependent endonuclease, which is an enzyme of the V-F type in the CRISPR system classification.

The terms "CasΦ" and "Cas12j" are used interchangeably and refer to a crRNA-dependent endonuclease, which belongs to the type V enzyme in the CRISPR system classification.

The term "PCR" refers to "polymerase chain reaction", which is a method for amplifying DNA fragments of interest in large quantities.

### Guide RNA-Reporter Nucleic Acid Complex Probe

As used herein, the terms "guide RNA-reporter nucleic acid complex probe of the present invention", "complex probe of the present invention" and "probe of the present invention" are used interchangeably and refer to the probes of the present invention that can be used to detect target nucleic acid molecules, including the guide RNA-reporter nucleic acid complex probe shown in Ia or Ib. It should be understood that the term also includes different forms of pairing, partial pairing or non-pairing between Z3 and Z5 in the complex probe. For example, Formula IIa is the state in which Z3 and Z5 form a pair in the complex probe of Formula Ia of the present invention.

In the present invention, it provides a novel guide RNA-reporter nucleic acid complex probe. A representative complex probe has the structure as shown in Formula Ia,

Z1-Z2-Z3-Z4-Z5 (Formula Ia)

wherein, Z1, Z2, Z3, Z4 and Z5 are as described above.

Another complex probe similar to Formula Ia is the structure of Formula Ib, in which Z1-Z2-Z3 and Z5 are two independent molecules: 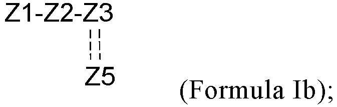 wherein, Z1, Z2, Z3, Z5 and " 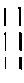 " are as described above.

Another complex probe similar to Formula Ia is the structure of Formula Ic,

Z5-Z4-Z3-Z2-Z1 (Formula Ic)

wherein, Z1, Z2, Z3, Z4 and Z5 are as described above.

Another complex probe similar to Formula Ia is the structure of Formula Id, in which Z1-Z2-Z3 and Z5 are two independent molecules: 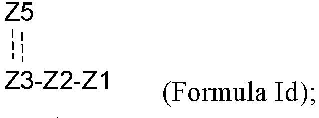 wherein, Z1, Z2, Z3, Z5 and " 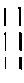 " are as described above.

In the present invention, the complex probe in the structure of Formula Ic or Formula Id is used in combination with Cas protein of type Cas13b.

In another preferred embodiment, the labels carried in the Z5 is a fluorescent group and a quenching group, and the fluorescent group and the quenching group are each independently located at the 5' end, 3' end and/or the middle of the nucleic acid probe.

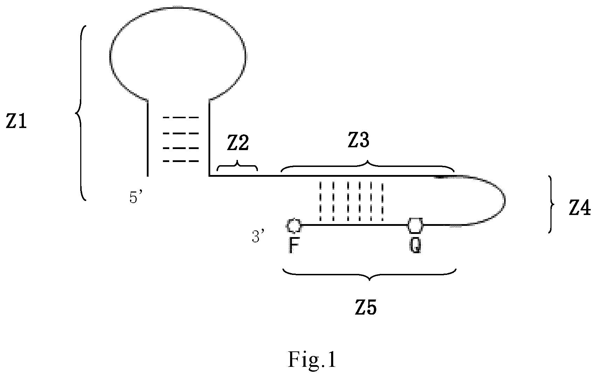

Taking Cas12 type as an example, the structure of a representative guide RNA-reporter nucleic acid complex probe is as shown in Figure 1. In the complex probe, a DNA sequence is added to the 3' end of the guide RNA, and a quenching group (Q) is added in the middle, and a fluorescent group (F) is added to the 3' end. The sequence of the complex probe acts as both a guide RNA and a fluorescent probe.

In order to facilitate understanding, taking Cas12 as an example, the following principles are provided for reference. However, it should be understood that the scope of the invention is not limited by this principle. In the present invention, in the absence of the target DNA, the end sequence (Z5) of DNA is complementary paired with a part of the sequence base (Z3) of the guide RNA to form a hairpin structure. When there is a specific target sequence, the complex probe of the present invention binds to the Cas12a protein and binds to the target sequence. At this time, the hairpin structure (that is, the pairing structure of Z3 and Z5 will be untied) will be opened, and the collateral single-stranded DNA cleavage activity of Cas12a will be activated at the same time, thereby cutting the DNA part of the complex probe (such as Z5), resulting in the fluorescent group separated from quenching group, in turn, the quenching group loses its quenching function and fluorescence is emitted.

In the present invention, even if multiple fluorescent probes are added to the same reaction system or detection system, since there is no free single-stranded DNA in the initial state, the Cas12 collateral cleavage activity activated by other targets does not shred the complex probes that do not bind to the target, nor does it interfere with each other.

### The reaction system of the invention

In the present invention, it provides a reaction system for detecting one or more (especially simultaneously detecting multiple) target nucleic acid molecules, which comprises:
(a) n kinds of guide RNA-reporter nucleic acid complex probes of the present invention (preferably, n is 2-500 or 2-200); and
(b) Cas protein, which is a Cas protein with collateral single-stranded nucleic acid cleavage activity.

The detection system provided by the present invention can detect m kinds of target nucleic acid molecules to be detected, wherein m is a positive integer, and m≤n.

In the present invention, the detection comprises: qualitative detection or quantitative detection.

In another embodiment of the present invention, the detection system further comprises:
(d1) polymerase for amplifying target DNA;
(d2) optional reverse transcriptase for reverse transcription;
(d3) optional transcription enzymes for transcription;
(d4) dNTPs for amplification reactions and/or reverse transcription reactions;
(d5) NTPs for transcription reactions.

Preferably, in the detection system provided by the present invention, the concentration of the target nucleic acid molecule to be detected in the system to be detected is 1 × 10⁻⁹ nM to 1 × 10³ nM; preferably 1 × 10⁻⁸ nM to 1 × 10² nM.

In another preferred embodiment, the concentration of the target nucleic acid molecule to be detected in the detection system is 1 to 1 x 10¹⁵ copies/ml, preferably 1 to 10¹⁰ copies/ml, more preferably 1 to 10⁵ copies/ml.

In another preferred embodiment, the concentration of the target nucleic acid molecule to be detected in the detection system is 1 to 1000 copies/ml, preferably 1 to 100 copies/ml, more preferably 1 to 10 copies/ml.

In another preferred embodiment, in the detection system, the molar ratio of each guide RNA-reporter nucleic acid complex probe to the corresponding target nucleic acid molecule is 1 : 1 to 10¹⁴ : 1, preferably 10 : 1 to 10⁵ : 1, more preferably 20 : 1 to 10³ : 1.

### The kit of the invention

In the present invention, it provides a kit for detecting one or more (especially simultaneously detecting multiple) target nucleic acid molecules, which comprises:
i) a first container and the n kinds of guide RNA-reporter nucleic acid complex probes as described herein located in the first container (preferably, n is a positive integer of 2-500 or 2-200);
ii) a second container and the Cas protein located in the second container, which is a Cas protein with collateral single-stranded nucleic acid cleavage activity;
iii) an optional third container and buffer located in the third container.

In a preferred embodiment, the detection system further comprises reagents for the nucleic acid amplification reaction. That is, in the detection system of the present invention, the target nucleic acid molecule can be amplified, and the amplified target nucleic acid molecules can be detected, which has the function of signal amplification.

In one embodiment of the present invention, the fourth container, the fifth container and the sixth container may be the same container or different containers. Preferably, two, more or all of the first to sixth containers may be the same container or different containers.

### Multiple detection

In the present invention, it provides a method for simultaneously detecting multiple target nucleic acid molecules in a sample, including the following steps:
(i) providing the detection system for detecting target nucleic acid molecules according to the first aspect of the present invention, and the detection system further contains a sample to be detected; and
(ii) detecting whether the guide RNA-reporter nucleic acid complex probe in the detection system is cleaved by the Cas protein, and the cleavage is a trans-cleavage of the collateral single-stranded nucleic acid;
wherein, if the guide RNA-reporter nucleic acid complex probe is cleaved by Cas protein, it means that there is a corresponding target nucleic acid molecule in the sample; and the guide RNA-reporter nucleic acid complex probe is not cleaved by Cas protein, it means that there is no corresponding target nucleic acid molecule in the sample.

In the present invention, the sample to be detected includes an unamplified sample and an amplified (or nucleic acid amplified) sample, and may also include an untranscribed sample and a transcribed sample.

In one embodiment of the present invention, the method of nucleic acid amplification is selected from the group consisting of PCR amplification, LAMP amplification, RPA amplification, ligase chain reaction, branched DNA amplification, NASBA, SDA, transcription-mediated amplification, rolling circle amplification, HDA, SPIA, NEAR, TMA and SMAP2.

In a preferred embodiment, the PCR comprises a high temperature PCR, a normal temperature PCR, and/or a low temperature PCR.

In a preferred embodiment of the present invention, the detection in step (ii) comprises a fluorescence detection assay. Preferably, the fluorescence detection assay is performed by using a microplate reader, or a fluorescence spectrophotometer.

In one embodiment of the present invention, the method is an *in vitro* detection method. Preferably, the sample is an *in vitro* or *ex vivo* sample.

In another embodiment of the present invention, the method is non-diagnostic and non-therapeutic.

### The main advantages of the present invention include:

1) High efficiency: The multi-target detection method of the present invention realizes that in the same detection system, a variety of simultaneous existed target nucleic acid molecules can be detected with extremely high sensitivity, and nucleic acid molecules (such as DNA) with a concentration of 10⁻¹⁷ M can be detected.
2) Low cost: Since multiple target nucleic acid molecules can be detected simultaneously in a small sample, the method of the present invention can greatly save the detection cost. There are no special materials or enzymes in the experiment, and there are few materials and reagents involved, so trace testing and analysis can be carried out.
3) Fast: When the test conditions are ready, it only takes about 1 hour from getting the sample to getting the test result.
4) Multipurpose: Different nucleic acid samples can be detected, including DNA samples and RNA samples.
5) Simple: There are no special and complicated steps. If the kit is made and the program is set, it is only necessary to simply add samples and other operations.

The present invention is further explained below in conjunction with specific example. It should be understood that these examples are only for illustrating the present invention and not intend to limit the scope of the present invention. The conditions of the experimental methods not specifically indicated in the following examples are usually in accordance with conventional conditions as described in Sambrook et al., Molecular Cloning: A Laboratory Manual (New York: Cold Spring Harbor Laboratory Press, 1989), or according to the conditions recommended by the manufacturer. Unless otherwise stated, percentages and parts are percentages by weight and parts by weight.

### Example 1: Cas12a multi-target detection test

### 1.1 Structure of Guide RNA-Reporter Nucleic Acid Complex Probe

Taking Cas12a type as an example, the structure of the guide RNA-reporter nucleic acid complex probe is as shown in Figure 1. In this example, two probes were designed and synthesized, and the sequences are as follows:

**Table 1 Exemplary Guide RNA-Reporter Nucleic Acid Complex Probe Sequences**

| Probe name | The underlined parts are RNA sequences, and the others are DNA sequences (5' to 3') | SEQ ID NO: |
|---|---|---|
| crRNA-DNMT 1-3-FAM-BHQ 1 | r(GAAUUUCUACUGUUGUAGAUcugauggucaauguc uguuacuc)ctc/BHQ1/taacagac-FAM | 1 |
| crRNA-sry-RO X-BHQ2 | r(AAUUUCUACUGUUGUAGAUucucugugcauggccu guaauuuc)CTTA/BHQ2/TTACAGGC-ROX | 2 |

| | | |
|---|---|---|
| *BHQ1 and BHQ2 are quenching groups; FAM and ROX are different fluorescent groups, respectively. | | |

### 1.2 Obtaining the Target DNA

First, it is necessary to amplify the target sequence, and PCR or any other amplification method can be used. In this embodiment, isothermal LAMP amplification is used.

LAMP amplification reaction: male saliva was heated at 95°C for 10 min and then used as a template. The total volume of each reaction system was 20 µL, and two types of primers were added to amplify gene DNMT1-3 on autosome and gene sry unique to male Y chromosome (see primer Table 2 for sequences), respectively. The specific primer amounts were 1.6 µM FIP and BIP, 0.2 µM F3 and B3, 0.4 µM LoopF and LoopB. The kit used for LAMP reaction was WarmStart^{®}LAMP Kit (NEB). The LAMP reaction procedure was 65°C for 40 min. The above products were called DNM and sry.

In addition, the genome of *Mycobacterium tuberculosis* was used as a template to amplify the IS6110-1 fragments. Its amplification products were called IS-1.

### 1.3 Cas12a reaction

In the 20 µL reaction system, adding

| | |
|---|---|
| 10*NEB buffer 3.1 | 2 µL |
| FnCas 12a | 1.5 µL (final concentration 1.5 µM after addition) |
| Probes | each added with 0.5 µL (3 kinds of probes of 10 µM) |
| Template | 0.5 µL (products of the LAMP amplification) |
| ddH₂O | added to 20 µL and mixed evenly. |

After the addition, it was detected in a fluorescence quantitative PCR instrument, and the reaction condition at a constant temperature of 40°C was used.

A total of 8 samples, the others were the same, only the templates added were different, which were 1. ddH₂O; 2. DNM; 3. IS-1; 4. sry; 5. DNM IS-1; 6. DNM sry; 7. IS-1 sry; 8. DNM IS-1 sry, respectively.

The results are shown in Figure 2, wherein Fig.2B is a Green channel, that is, detecting FAM fluorescence for DNM target, in which samples 2, 5, 6 and 8 can be detected with significant rising curves respectively; Fig.2C is an Orange channel, that is, detecting ROX fluorescence for sry target, in which samples 4, 6, 7 and 8 can be detected significant rising curves respectively.

The above results show that once there is a corresponding target in the detection system, the corresponding fluorescence signal can be detected, which is consistent with the expected result; in turn, the existence of a certain target can be judged according to the type of the detected fluorescent signal.

**Table 2 Primer Sequences**

| Primer name | Sequence (5' to 3') | SEQ ID NO: |
|---|---|---|
| LAMP-DNM-F3 | gtgaacgttcccttagcact | 3 |
| LAMP-DNM-B3 | gggagggcagaactagtcc | 4 |
| LAMP-DNM-FIP | cgccacttgacaggcgagtaactgccacttattgggtcagc | 5 |
| LAMP-DNM-BIP | gcgtgttccccagagtgacttagcagcttcctcctcctt | 6 |
| LAMP-DNM-Loop F | aggaaacattaacgtactgatg | 7 |
| LAMP-DNM-Loop B | ttccttttatttcccttcagc | 8 |
| LAMP-sry-F3 | tctctgtgcatggcctgta | 9 |
| LAMP-sry-B3 | aacagtaaaggcaacgtcca | 10 |
| LAMP-sry-FIP | gcagctgggataccagtggaagtgcctcctggaagaatgg | 11 |
| LAMP-sry-BIP | tctctagagccatcttgcgcctgaagcgacccatgaacgc | 12 |
| LAMP-sry-LoopF | tgcttactgaagccgaaaaatg | 13 |
| LAMP-sry-LoopB | tgatcgcgagaccacacgatg | 14 |
| IS6110-1-F3 | CGCCGCCAACTACGGT | 15 |
| IS6110-1-B3 | CGGCGCTGGACGAGAT | 16 |
| IS6110-1-FIP | GCATCTGGCCACCTCGATGCCTTACGGTG CCCGCAAAGT | 17 |
| IS6110-1-BIP | ACGGCTGATGACCAAACTCGGCGGCTGTG GCCGGATCA | 18 |
| IS6110-1-loopF | TCACGGTTCAGGGTTAGC | 19 |
| IS6110-1-loopR | AAGCCCGCAGGACCACGATC | 20 |

### Example 2: Cas12b Multi-Target Detection Test

### 2.1 Structure of Cas12b reporter nucleic acid complex probe

### 2.1.1 Synthesis of Guide RNA-Reporter Nucleic Acid Complex Probe

Taking the AacCas12b type as an example, the structure of the guide RNA-reporter nucleic acid complex probe is as shown in Figure 6 and consists of a tracrRNA and a crRNA reporter nucleic acid probe. In this example, two probes were designed and synthesized, and the sequences are as follows:

**Table 3 Exemplary Guide RNA-Reporter Nucleic Acid Complex Probe Sequences**

| Probe name | The underlined parts are RNA sequences, and the others are DNA sequences (5' to 3') | SEQ ID NO: |
|---|---|---|
| crRNA-SE-5-ROX-BHQ2 | r(AAUUUCUACUGUUGUAGAUugaacuuuauuggcgg uauuucg)GC/BHQ2/TAATACCGC-ROX | 21 |
| crRNA-0157-6-FAM-BHQ1 | r(AAUUUCUACUGUUGUAGAUcacuaccaccaaauaac uggucg)GC/BHQ1/TCCAGTTAT-FAM | 22 |

| | | |
|---|---|---|
| *BHQ1 and BHQ2 are quenching groups; FAM and ROX are different fluorescent groups, respectively. | | |

### 2.1.2 Preparation of Cas12b tracrRNA:

First, a transcription template was prepared by annealing a T7-crRNA-F with a synthetic oligonucleotide Cas12b_tracrRNA (Table 3). Specifically, the paired oligonucleotides (4 µM) were annealed in 1×PCR buffer (Transgen Biotech) with a total volume of 50 µL, followed by an annealing procedure: initial denaturation at 95°C for 5 minutes, then cooling from 95°C to 20°C, using a thermal cycler to decrease by 1°C per minute. The tracrRNA was synthesized using a T7 high-yield transcription kit, and the reaction was carried out overnight (about 16h) at 37°C. Template DNA was treated with DNase I, then RNA was purified with RNA purification and concentration kit, quantified with NanoDrop 2000C, and stored in a refrigerator at -80°C.
Note: Cas12b_tracrRNA (SEQ ID NO: 23): 
5’-TTTTTGAGAAGCTCAACGGGCTTTGCCACCTGGAAAGTGGCCATT
GGCACACCCGTTGAAAAATTCTGTCCTCTAGACCCTATAGTGAGTCGTAT
TA-3’;
T7-crRNA-F (SEQ ID NO: 24): 5'-GAAATTAATACGACTCACTATAGGG-3'

### 2.1.3 Annealing reaction of Cas12b tracrRNA: crRNA-reporter nucleic acid complex probe:

Under Tris buffer conditions (50 mM Tris-HCl [pH 8.3],75 mM KCl, 3 mM MgCl₂), the tracrRNA : crRNA-reporter nucleic acid complex probe were mixed at a molar concentration of 2:1 (final concentrations of 10 µM and 5 µM, respectively), and the annealing reaction was carried out on a PCR instrument. Initial denaturation at 85°C for 5 minutes, then cooling from 85°C to 25°C, using a thermal cycler to decrease by 3°C per minute. The annealed complex probe can be used in Cas12b cleavage reaction to detect target nucleic acid.

### 2.2 Obtaining the Target DNA

First, it is necessary to amplify the target sequence, and PCR or any other amplification method can be used. In this embodiment, PCR amplification is used.

PCR reaction: *Salmonella* genomic DNA and *Escherichia coli* 0157 genomic DNA were extracted as templates for PCR amplification. The total volume of each reaction system was 20 µL, and two types of primers were added respectively to amplify the specific fragments in *Salmonella* (product was named SE) and the specific *fragments* in *Escherichia coli* 0157 (product was named 0157) (see primer Table 4 for sequences). The PCR reaction procedure was 95°C for 2min, and then 35 cycles were started at 98°C for 10s, 60°C for 15s, and 72°C for 10s. After PCR was completed, its products were used directly for the Cas12b reaction.

### 2.3 Cas12b reaction

In the 20 µL reaction system, adding

| | |
|---|---|
| 10*NEB buffer 3.1 | 2 µL |
| AacCas12b | 1.5 µL (final concentration 1.5 µM after addition) |
| Complex probe | 1 µL (the final concentration of each probe is 500 nM) |
| ddH₂O | added to 19.5 µL and mixed evenly. |

After addition, it was placed at 48°C for 5 min, then 0.5 µL of template (product after PCR amplification) was added to the reaction system, and the reaction system was placed in a fluorescence quantitative PCR instrument for detection. FAM fluorescence was detected under a constant temperature of 48°C.

There were 2 reaction samples in total. The difference was that the templates added were different. They were the PCR amplification products of SE and 0157, respectively. The reaction system with sterile water was used as a negative control.

The fluorescence quantitative PCR instrument used in this reaction is ABI StepOne Plus, and the detected signal is FAM fluorescence. The results are shown in Figure 7. After deducting the background signal (that is, adding sterile water as a template), the FAM fluorescence signal of the reaction group with SE template was very low, while the FAM fluorescence signal of the reaction group with 0157 template increased rapidly, which was significantly different from the signal intensity of SE group. Based on the above results, it can be inferred: The SE template cannot activate the cleavage of the 0157 reporter nucleic acid probe, while the 0157 template can activate the cleavage of the 0157 reporter nucleic acid probe. On the contrary, the results of ROX fluorescence detection by microplate reader show that 0157 template cannot activate the cleavage of the SE reporter nucleic acid probe, while the SE template can activate the cleavage of the SE reporter nucleic acid probe. The above results show that once there is a corresponding target in the detection system, the corresponding fluorescence signal can be detected, which is consistent with the expected result; in turn, the existence of a certain target can be judged according to the type of the detected fluorescent signal.

**Table 4 Primer Sequences**

| Primer name | Sequence (5'-3') | SEQ ID NO: |
|---|---|---|
| SE-F | TGTCACCGTGGTCCAGTTTA | 25 |
| SE-R | CGACAAGACCATCACCAATG | 26 |
| O157-F3 | gatggga a cgattatatcgaagg | 27 |
| 0157-R2 | cctgacagaatattataagctccg | 28 |

### Example 3: Cas14 Multi-Target Detection Test

### 3.1 Structure of Cas14 reporter nucleic acid complex probe

### 3.1.1 Synthesis of Guide RNA-Reporter Nucleic Acid Complex Probe

Taking Cas14a1 as an example, the structure of the guide RNA-reporter nucleic acid complex probe is as shown in Figure 6 and consists of a tracrRNA and a crRNA reporter nucleic acid probe. In this example, two probes were designed and synthesized, and the sequences are as follows:

**Table 5 Exemplary Guide RNA-Reporter Nucleic Acid Complex Probe Sequences**

| Probe name | The underlined parts are RNA sequences, and the others are DNA sequences (5' to 3') | SEQ ID NO: |
|---|---|---|
| Cas14-SE-PAM5 -ROX-BHQ2 | r(GACGAAUGAAGGAAUGCAACugaacuuuauugg cgguauu)TT/BHQ2/TACCGCCA | 29 |
| Cas14-O157-PA M6-FAM-BHQ1 | r(GACGAAUGAAGGAAUGCAACcacuaccaccaaa uaacugg)GG/BHQ1/TGTTATTTG | 30 |

| | | |
|---|---|---|
| *BHQ1 and BHQ2 are quenching groups; FAM and ROX are different fluorescent groups, respectively. | | |

### 3.1.2 Preparation of Cas14a1 tracrRNA:

First, a fragment of tracrRNA with Cas14a1 was synthesized and cloned onto the pUC57 vector. Then T7-crRNA-F and Cas14a-tracr-R primers (Table 6) were used for amplification, and the amplified product was purified for transcription of tracrRNA. TracrRNA was synthesized using 200ng transcription template and T7 high-yield transcription kit, and the reaction was carried out at 37°C overnight (about 16h). Template DNA was treated with DNase I, then RNA was purified with RNA purification and concentration kit, quantified with NanoDrop 2000C, and stored in a refrigerator at -80°C.
Note: Cas14a-tracr-R (SEQ ID NO: 31):
   5'- AAATGAATTTGTTTCGAGGGTTAC -3'
Synthetic Cas14a1 tracrRNA sequence with T7 promoter (SEQ ID NO: 32):
   5'-**GAAATTAATACGACTCACTATAGGG**CTTCACTGATAAAGTGGAG AACCGCTTCACCAAAAGCTGTCCCTTAGGGGATTAGAACTTGAGTGAAGG TGGGCTGCTTGCATCAGCCTAATGTCGAGAAGTGCTTTCTTCGGAAAGTA ACCCTCGAAACAAATTCATTTTTC-3', the sequence marked in bold is the T7 promoter sequence, and the remaining sequence is the Cas14a1 tracrRNA sequence.

### 3.1.3 Annealing reaction of Cas14 tracrRNA: crRNA-reporter nucleic acid complex probe:

Under Tris buffer conditions (50 mM Tris-HCl [pH 8.3], 75 mM KCl, 3 mM MgCl2), the tracrRNA: crRNA-reporter nucleic acid complex probes were mixed at a molar concentration of 2:1 (final concentrations of 10 µM and 5 µM, respectively), and the annealing reaction was carried out on a PCR instrument. Initial denaturation at 85°C for 5 minutes, then cooling from 85°C to 25°C, using a thermal cycler to decrease by 3°C per minute. The annealed complex probe can be used in Cas14 cleavage reaction to detect target nucleic acid.

### 3.2 Obtaining the Target DNA

First, it is necessary to amplify the target sequence, and PCR or any other amplification method can be used. In this embodiment, PCR amplification is used.

PCR reaction: *Salmonella* genomic DNA and *Escherichia coli* 0157 genomic DNA were extracted as templates for PCR amplification. The total volume of each reaction system was 20 µL, and two types of primers were added for amplification. In each pair of primers, the 5' end of one of them is modified by phosphorothioate. The above primer pairs were used to amplify the specific fragment in Salmonella (product named SE-ps) and the specific fragment in Escherichia coli 0157 (product named O157-ps) respectively (see primer Table 6 for sequences). The PCR reaction procedure was 95°C for 2 min, and then 35 cycles were started at 98°C for 10s, 60°C for 15s, and 72°C for 10s. After PCR was completed, its products were used directly for the Cas14 reaction.

### 3.3 Cas14 reaction

In the 20 µL reaction system, adding:

| | |
|---|---|
| 10* Cas14 buffer | 2 µL |
| Cas14a1 | 1.5 µL (final concentration 1.5 µM after addition) |
| Complex probe | 1 µL (the final concentration of each probe is 500 nM) |
| ddH₂O | added to 19 µL and mixed evenly. |

After addition, it was placed at 37°C for 5 min, then 0.5 µL of template (product after PCR amplification) and 0.5 µL T7 exonuclease (10 Units/µL) were added to the reaction system, and the reaction system was placed in a fluorescence quantitative PCR instrument for detection. FAM fluorescence was detected under a constant temperature of 37°C. The reaction buffer of 10*Cas14 is: 250mM NaCl, 200mM HEPES, pH 7.5, 10mM DTT, 50% glycerol and 50mM MgCl₂.

There were 2 reaction samples in total. The difference was that the templates added were different. They were the PCR amplification products of SE-ps and O157-ps, respectively. The reaction system with sterile water was used as a negative control.

The fluorescence quantitative PCR instrument used in this reaction is ABI StepOne Plus, and the detected signal is FAM fluorescence. The results are shown in Figure 8. After deducting the background signal (that is, adding sterile water as a template), the FAM fluorescence signal of the reaction group with SE-ps template was lower, while the FAM fluorescence signal of the reaction group with O157-ps template increased rapidly, which was significantly different from the signal intensity of SE-ps group. Based on the above results, it can be inferred: The SE-ps template cannot activate the cleavage of the O157-ps reporter nucleic acid probe, while the O157-ps template can activate the cleavage of the O157-ps reporter nucleic acid probe. On the contrary, the results of ROX fluorescence detection by microplate reader show that O157-ps template cannot activate the cleavage of the SE-ps reporter nucleic acid probe, while the SE-ps template can activate the cleavage of the SE-ps reporter nucleic acid probe. The above results show that once there is a corresponding target in the detection system, the corresponding fluorescence signal can be detected, which is consistent with the expected result; in turn, the existence of a certain target can be judged according to the type of the detected fluorescent signal.

**Table 6 Primer Sequences**

| Primer name | Sequence (5'-3') | SEQ ID NO: |
|---|---|---|
| SE-F | tgtcaccgtggtccagttta | 25 |
| SE-R-ps | c*g*a*c*aagaccatcaccaatg | 33 |
| O157-F-ps | c*a*g*t*agggaagcgaacagag | 34 |
| 0157-R2 | cctgacagaatattataagctccg | 28 |

| | | |
|---|---|---|
| Note: Except for phosphorothioate modification (marked with *), the remaining sequences are consistent with the amplification primers of Cas12b. | | |

## Claims

1. A detection system for detecting target nucleic acid molecules, which comprises:
(a) n kinds of guide RNA-reporter nucleic acid complex probes, which has a structure as shown in Formula Ia, Ib, Ic or Id,
Z1-Z2-Z3-Z4-Z5 (Formula Ia) 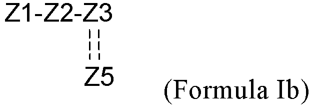
Z5-Z4-Z3-Z2-Z1 (Formula Ic) 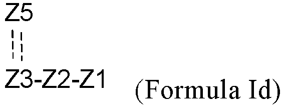
wherein,
Z1 is a first stem-loop structure region;
Z2 is none or a nucleic acid linker region;
Z3 is a guide RNA region, wherein the Z3 comprises a nucleic acid sequence that can guide the Cas protein to specifically bind to a target nucleic acid molecule;
Z5 is a single-stranded nucleic acid to be cleaved with a detectable label, wherein the detectable label presents a different detection state when the single-stranded nucleic acid to be cleaved is cleaved and not cleaved, thereby being detected;
wherein, when the target nucleic acid does not exist in the detection system, Z3 and Z5 form a complementary paired double-stranded structure region, and when the target nucleic acid exists in the detection system, Z3 and Z5 do not form the complementary paired double-stranded structure region;
Z4 is none, or a chemical bond or a linker region used for connecting Z3 and Z5;
" 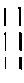 " is a hydrogen bond for complementary base pairing;
and, n is a positive integer with n ≥ 1; and
(b) Cas protein, which is a Cas protein with collateral single-stranded nucleic acid cleavage activity.

2. The detection system of claim 1, wherein the Cas protein is selected from the group consisting of Cas12 type, Cas13a type, Cas13b type, Cas14 type, and a combination thereof.

3. The detection system of claim 1, wherein when the target nucleic acid does not exist in the detection system, the guide RNA-report nucleic acid complex probe is of Formula IIa structure: 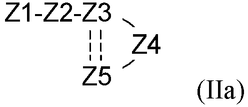 wherein,
Z1, Z2, Z3, Z4 and Z5 are as described above,
" 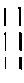 " is a hydrogen bond for complementary base pairing.

4. The detection system of claim 1, wherein when the target nucleic acid does not exist in the detection system, the guide RNA-report nucleic acid complex probe is of Formula IIc structure: 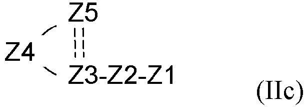
wherein,Z1, Z2, Z3, Z4 and Z5 are as described above,
" 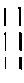 " is a hydrogen bond for complementary base pairing.

5. The detection system of claim 1, wherein the length of the Z1 is 10-300nt, preferably 19-100nt, more preferably 19-91nt.

6. The detection system of claim 1, wherein the Z2 is a nucleic acid linker region with a length of 0-20nt.

7. The detection system of claim 1, wherein:
(i) n≥ 2, and n is a positive integer;
(ii) the detectable label is a fluorescent group, and Z5 has a fluorescent group, while Z4 and/or Z5 also has a quenching group, and the fluorescent signal emitted by the fluorescent group can be detected when and only when the single-stranded nucleic acid to be cleaved is cleaved; and/or
(iii) among the n kinds of guide RNA-reporter nucleic acid complex probes, the fluorescent groups are different from each other, so that they can be distinguished.

8. The detection system of claim 1, wherein the length of the Z3 is 15-50nt, preferably 16-40nt, more preferably 16-34nt.

9. The detection system of claim 1, wherein the Z5 is a DNA single-stranded nucleic acid sequence, or an RNA single-stranded nucleic acid sequence, or a nucleic acid sequence having both RNA and DNA.

10. The detection system of claim 1, wherein the length of the Z5 is 3-50nt, preferably 4-30nt, more preferably 6-12nt.

11. The detection system of claim 1, wherein the detection comprises: a qualitative detection or a quantitative detection.

12. A kit for detecting target nucleic acid molecules, which comprises:
i) a first container and the n kinds of guide RNA-reporter nucleic acid complex probes with the structures as shown in Formula Ia, Ib, Ic or Id located in the first container,
Z1-Z2-Z3-Z4-Z5 (Formula Ia) 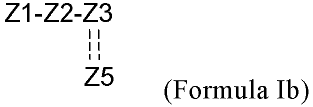
Z5-Z4-Z3-Z2-Z1 (Formula Ic) 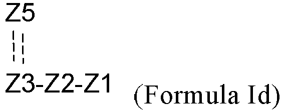
wherein,
Z1 is a first stem-loop structure region;
Z2 is none or a nucleic acid linker region;
Z3 is a guide RNA region, wherein the Z3 comprises a nucleic acid sequence that can guide the Cas protein to specifically bind to a target nucleic acid molecule;
Z5 is a single-stranded nucleic acid to be cleaved with a detectable label, wherein the detectable label presents a different detection state when the single-stranded nucleic acid to be cleaved is cleaved and not cleaved, thereby being detected;
wherein, when the target nucleic acid does not exist in the detection system, Z3 and Z5 form a complementary paired double-stranded structure region, and when the target nucleic acid exists in the detection system, Z3 and Z5 do not form the complementary paired double-stranded structure region;
Z4 is none, or a chemical bond or a linker region used for connecting Z3 and Z5;
" 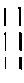 " is a hydrogen bond for complementary base pairing;
and, n is a positive integer with n ≥ 1;
ii) a second container and the Cas protein located in the second container, which is a Cas protein with collateral single-stranded nucleic acid cleavage activity;
iii) an optional third container and buffer located in the third container.

13. The kit of claim 12, wherein the kit further comprises:
iv) a fourth container and the polymerase for amplifying the target DNA located in the fourth container;
v) an optional fifth container and the reverse transcriptase for reverse transcription and/or the transcriptase for transcription located in the fifth container;
vii) a sixth container and the dNTPs for amplification reactions and/or reverse transcription reactions and/or NTPs for transcription reactions located in the sixth container.

14. A method for detecting target nucleic acid molecules in a sample, which comprises the following steps:
(i) providing the detection system for simultaneously detecting multiple target nucleic acid molecules of claim 1, and the detection system further contains a sample to be detected; and
(ii) detecting whether the guide RNA-reporter nucleic acid complex probe in the detection system is cleaved by the Cas protein, and the cleavage is a collateral (or trans) cleavage for the single-stranded nucleic acid;
wherein, if the guide RNA-reporter nucleic acid complex probe is cleaved by the Cas protein, it means that there is a corresponding target nucleic acid molecule in the sample; and if the guide RNA-reporter nucleic acid complex probe is not cleaved by the Cas protein, it means that there is no corresponding target nucleic acid molecule in the sample.

15. The method of claim 14, wherein the sample to be analysed is an amplified sample.

## Patentansprüche

1. Detektionssystem zum Detektieren von Ziel-Nukleinsäuremolekülen, wobei es Folgendes umfasst:
(a) n Arten von Sonden aus einem Komplex von Guide-RNA - Reporter-Nukleinsäure, welcher eine Struktur gemäß der Darstellung in Formel Ia, Ib, Ic oder Id hat,
Z1-Z2-Z3-Z4-Z5 (Formel Ia) 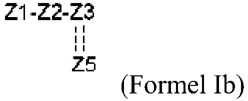
Z5-Z4-Z3-Z2-Z1 (Formel Ic) 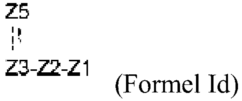
wobei,
Z1 eine erste Region mit Stamm-Schleifen-Struktur ist;
Z2 fehlt oder eine Nukleinsäuren-Linkerregion ist;
Z3 eine Guide-RNA-Region ist, wobei Z3 eine Nukleinsäuresequenz umfasst, die das Cas-Protein derart steuern kann, dass es auf spezifische Weise an ein Ziel-Nukleinsäuremolekül bildet;
Z5 eine zu spaltende Einzelstrang-Nukleinsäure mit einem detektierbaren Marker ist, wobei der detektierbare Marker einen unterschiedlichen Detektionszustand aufweist, je nachdem, ob die Einzelstrang-Nukleinsäure gespalten oder nicht gespalten ist, wobei sie auf diese Weise detektiert wird;
wobei, wenn die Zielnukleinsäure in dem Detektionssystem nicht vorliegt, Z3 und Z5 eine Region mit komplementär gepaarter Doppelstrang-Struktur bilden, und Z3 und Z5 keine Region mit komplementär gepaarter Doppelstrang-Struktur bilden, wenn die Zielnukleinsäure in dem Detektionssystem vorliegt,
Z4 fehlt oder für eine chemische Bindung oder eine Linkerregion steht, die dazu verwendet wird, Z3 und Z5 zu verbinden;
" 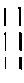 " eine Wasserstoffbrückenbindung für die Paarung komplementärer Basen ist;
und n eine positive ganze Zahl ist, wobei n ≥ 1; und
(b) Cas-Protein, wobei es sich um ein Cas-Protein mit einer Wirkung des kollateralen Spaltens von Einzelstrang-Nukleinsäuren handelt.

2. Detektionssystem nach Anspruch 1, wobei das Cas-Protein aus der Gruppe ausgewählt ist, die aus Cas-Typ 12, Cas-Typ 13a, Cas-Typ 13b, Cas-Typ 14 und einer Kombination davon besteht.

3. Detektionssystem nach Anspruch 1, wobei, wenn die Zielnukleinsäure in dem Detektionssystem nicht vorliegt, die Sonde aus einem Komplex von Guide-RNA - Reporter-Nukleinsäure eine Struktur nach Formel IIa aufweist: 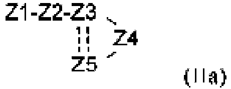 wobei
Z1, Z2, Z3, Z4 und Z5 der obigen Beschreibung entsprechen,
" 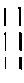 " eine Wasserstoffbrückenbindung für die Paarung komplementärer Basen ist.

4. Detektionssystem nach Anspruch 1, wobei, wenn die Zielnukleinsäure in dem Detektionssystem nicht vorliegt, die Sonde aus einem Komplex von Guide-RNA - Reporter-Nukleinsäure eine Struktur nach Formel IIc aufweist 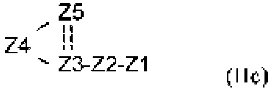
wobei Z1, Z2, Z3, Z4 und Z5 der obigen Beschreibung entsprechen,
" 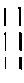 " eine Wasserstoffbrückenbindung für die Paarung komplementärer Basen ist.

5. Detektionssystem nach Anspruch 1, wobei Z1 eine Länge von 10 bis 300 nt, vorzugsweise 19 bis 100 nt, insbesondere 19 bis 91 nt hat.

6. Detektionssystem nach Anspruch 1, wobei es sich bei Z2 um eine Nukleinsäure-Linkerregion mit einer Länge von 0 bis 20 nt handelt.

7. Detektionssystem nach Anspruch 1, wobei:
(i) n ≥ 2, und n eine positive ganze Zahl ist;
(ii) es sich bei dem detektierbaren Marker um eine fluoreszierende Gruppe handelt und Z5 eine fluoreszierende Gruppe aufweist, wohingegen Z4 und/oder Z5 weiterhin eine auslöschende Gruppe aufweisen und das Fluoreszenzsignal, welches von der fluoreszierenden Gruppe ausgesendet wird, dann und nur dann detektiert werden kann, wenn die zu spaltende Einzelstrang-Nukleinsäure gespalten ist; und/oder
(iii) bei den n Arten von Sonden aus einem Komplex von Guide-RNA - Reporter-Nukleinsäure sich die fluoreszierenden Gruppe jeweils andersartig als bei den übrigen sind, sodass diese unterschieden werden können.

8. Detektionssystem nach Anspruch 1, wobei Z3 eine Länge von 15 bis 50 nt, vorzugsweise 16 bis 40 nt, insbesondere 16 bis 34 nt aufweist.

9. Detektionssystem nach Anspruch 1, wobei es sich bei Z5 um eine Einzelstrang-DNA-Nukleinsäuresequenz oder eine Einzelstrang-RNA-Nukleinsäuresequenz oder um eine Nukleinsäuresequenz handelt, die sowohl RNA als auch DNA aufweist.

10. Detektionssystem nach Anspruch 1, wobei Z5 eine Länge von 3 bis 50 nt, vorzugsweise 4 bis 30 nt, insbesondere 6 bis 12 nt aufweist.

11. Detektionssystem nach Anspruch 1, wobei die Detektion Folgendes umfasst: eine qualitative Detektion oder eine quantitative Detektion.

12. Kit zur Detektion von Ziel-Nukleinsäuremolekülen, wobei es Folgendes umfasst:
i) einen ersten Behälter und die n Arten von Sonden aus einem Komplex von Guide-RNA - Reporter-Nukleinsäure gemäß der Darstellung in Formel Ia, Ib, Ic oder Id, wobei diese sich in dem ersten Behälter befinden,
Z1-Z2-Z3-Z4-Z5 (Formel Ia) 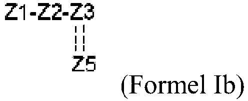
Z5-Z4-Z3-Z2-Z1 (Formel Ic) 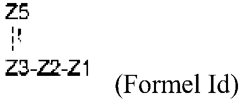
wobei
Z1 eine erste Region mit Stamm-Schleifen-Struktur ist;
Z2 fehlt oder eine Nukleinsäuren-Linkerregion ist;
Z3 eine Guide-RNA-Region ist, wobei Z3 eine Nukleinsäuresequenz umfasst, die das Cas-Protein derart steuern kann, dass es auf spezifische Weise an ein Ziel-Nukleinsäuremolekül bildet;
Z5 eine zu spaltende Einzelstrang-Nukleinsäure mit einem detektierbaren Marker ist, wobei der detektierbare Marker einen unterschiedlichen Detektionszustand aufweist, je nachdem, ob die Einzelstrang-Nukleinsäure gespalten oder nicht gespalten ist, wobei sie auf diese Weise detektiert wird;
wobei, wenn die Zielnukleinsäure in dem Detektionssystem nicht vorliegt, Z3 und Z5 eine Region mit komplementär gepaarter Doppelstrang-Struktur bilden, und Z3 und Z5 keine Region mit komplementär gepaarter Doppelstrang-Struktur bilden, wenn die Zielnukleinsäure in dem Detektionssystem vorliegt,
Z4 fehlt oder für eine chemische Bindung oder eine Linkerregion steht, die dazu verwendet wird, Z3 und Z5 zu verbinden;
" 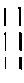 " eine Wasserstoffbrückenbindung für die Paarung komplementärer Basen ist;
und n eine positive ganze Zahl ist, wobei n ≥ 1; und
ii) einen zweiten Behälter und das Cas-Protein, welches sich in dem zweiten Behälter befindet, wobei es sich um ein Cas-Protein mit einer Wirkung des kollateralen Spaltens von Einzelstrang-Nukleinsäuren handelt,
iii) einen optionalen dritten Behälter und einen Puffer, der sich in dem dritten Behälter befindet.

13. Kit nach Anspruch 12, wobei das Kit weiterhin Folgendes umfasst:
iv) einen vierten Behälter und die Polymerase zur Vervielfältigung der Ziel-DNA, die sich in dem vierten Behälter befindet;
v) einen optionalen fünften Behälter und die reverse Transkriptase für eine reverse Transkription und/oder die Transkriptase für eine Transkription, wobei diese sich in dem fünften Behälter befindet;
vii) einen sechsten Behälter und die sNTP für Vervielfältigungsreaktionen und/oder reverse Transkriptionsreaktionen und/oder NTP für Transkriptionsreaktionen, wobei diese sich in dem sechsten Behälter befinden.

14. Verfahren zum Detektieren von Ziel-Nukleinsäuremolekülen in einer Probe, wobei es die folgenden Schritte umfasst:
(i) Bereitstellen des Detektionssystems zum gleichzeitigen Detektieren mehrerer Ziel-Nukleinsäuremoleküle nach Anspruch 1, wobei das Detektionssystem weiterhin eine zu detektierende Probe enthält; und
(ii) Detektieren, ob die Sonde aus einem Komplex von Guide-RNA - Reporter-Nukleinsäure von dem Cas-Protein gespalten wird und es sich bei der Spaltung um eine kollaterale (oder trans-) Spaltung für die Einzelstrang-Nukleinsäure handelt;
wobei, wenn die Sonde aus einem Komplex von Guide-RNA - Reporter-Nukleinsäure von dem Cas-Protein gespalten wird, dies bedeutet, dass in der Probe ein entsprechendes Ziel-Nukleinsäuremolekül vorliegt; und wenn die Sonde aus einem Komplex von Guide-RNA - Reporter-Nukleinsäure nicht von dem Cas-Protein gespalten wird, dies bedeutet, dass in der Probe keinerlei entsprechendes Ziel-Nukleinsäuremolekül vorliegt.

15. Verfahren nach Anspruch 14, wobei es sich bei der zu analysierenden Probe um eine vervielfältigte Probe handelt.

## Revendications

1. Système de détection pour détecter des molécules d'acide nucléique cibles, qui comprend :
(a) n types de sondes de complexe d'acide nucléique rapporteur d'ARN guide, présentant une structure telle que représentée dans les formules Ia, Ib, Ic ou Id,
Zl-Z2-Z3-Z4-Z5 (Formule Ia) 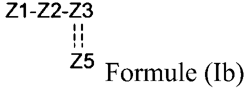
Z5-Z4-Z3-Z2-Z1 (Formule Ic) 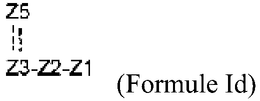
dans lequel,
Z1 est une première région de structure tige-boucle ;
Z2 est absent ou une région de liaison d'acide nucléique ;
Z3 est une région d'ARN guide, dans lequel la région Z3 comprend une séquence d'acide nucléique qui peut guider la protéine Cas pour se lier spécifiquement à une molécule d'acide nucléique cible ;
Z5 est un acide nucléique simple brin clivable avec un marqueur détectable, dans lequel le marqueur détectable présente un état de détection différent lorsque l'acide nucléique simple brin clivable est clivé et non clivé, étant ainsi détecté ;
dans lequel, lorsque l'acide nucléique cible n'existe pas dans le système de détection, Z3 et Z5 forment une région de structure double brin appariée complémentaire, et lorsque l'acide nucléique cible existe dans le système de détection, Z3 et Z5 ne forment pas la région de structure double brin appariée complémentaire ;
Z4 est absent, ou une liaison chimique ou une région de liaison utilisée pour connecter Z3 et Z5 ;
« 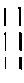 » est une liaison hydrogène pour l'appariement de bases complémentaires ;
et, n est un entier positif avec n ≥ 1 ; et
(b) la protéine Cas, qui est une protéine Cas avec une activité collatérale de clivage d'acide nucléique simple brin.

2. Système de détection selon la revendication 1, dans lequel la protéine Cas est choisie dans le groupe consistant en le type Cas 12, le type Cas13a, le type Cas13b, le type Cas14, et une combinaison de ceux-ci.

3. Système de détection selon la revendication 1, dans lequel lorsque l'acide nucléique cible n'existe pas dans le système de détection, la sonde de complexe d'acide nucléique rapporteur d'ARN guide est de structure de formule Ila : 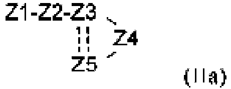 dans lequel,
Z1, Z2, Z3, Z4 et Z5 sont tels que décrits ci-dessus,
« 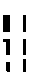 » est une liaison hydrogène pour l'appariement de bases complémentaires.

4. Système de détection selon la revendication 1, dans lequel lorsque l'acide nucléique cible n'existe pas dans le système de détection, la sonde de complexe d'acide nucléique rapporteur d'ARN guide est de structure de formule IIc : 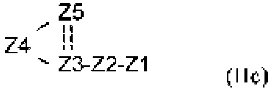
dans lequel, Z1, Z2, Z3, Z4 et Z5 sont tels que décrits ci-dessus,
« 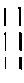 » est une liaison hydrogène pour l'appariement de bases complémentaires.

5. Système de détection selon la revendication 1, dans lequel la longueur de Z1 est de 10 à 300 nt, de préférence de 19 à 100 nt, de manière encore préférée de 19 à 91 nt.

6. Système de détection selon la revendication 1, dans lequel Z2 est une région de liaison d'acide nucléique présentant une longueur de 0 à 20 nt.

7. Système de détection selon la revendication 1, dans lequel :
(i) n ≥ 2, et n est un entier positif ;
(ii) le marqueur détectable est un groupe fluorescent, et Z5 présente un groupe fluorescent, tandis que Z4 et/ou Z5 présentent également un groupe d'extinction, et le signal fluorescent émis par le groupe fluorescent peut être détecté lorsque et uniquement lorsque l'acide nucléique simple brin clivable est clivé ; et/ou
(iii) parmi les n types de sondes de complexe d'acide nucléique rapporteur d'ARN guide, les groupes fluorescents sont différents les uns des autres, de sorte qu'ils peuvent être distingués.

8. Système de détection selon la revendication 1, dans lequel la longueur de Z3 est de 15 à 50 nt, de préférence de 16 à 40 nt, de manière encore préférée de 16 à 34 nt.

9. Système de détection selon la revendication 1, dans lequel Z5 est une séquence d'acide nucléique simple brin d'ADN, ou une séquence d'acide nucléique simple brin d'ARN, ou une séquence d'acide nucléique présentant à la fois de l'ARN et de l'ADN.

10. Système de détection selon la revendication 1, dans lequel la longueur de Z5 est de 3 à 50 nt, de préférence de 4 à 30 nt, de manière encore préférée de 6 à 12 nt.

11. Système de détection selon la revendication 1, dans lequel la détection comprend : une détection qualitative ou une détection quantitative.

12. Kit pour détecter des molécules d'acide nucléique cibles, qui comprend :
i) un premier récipient et les n types de sondes de complexe d'acide nucléique rapporteur d'ARN guide avec les structures telles que représentées dans la formule Ia, Ib, Ic ou Id situés dans le premier récipient,
Z1-Z2-Z3-Z4-Z5 (Formule Ia) 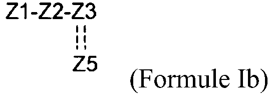
Z5-Z4-Z3-Z2-Z1 (Formule Ic) 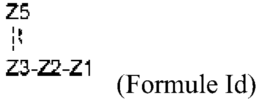
dans lequel,
Z1 est une première région de structure tige-boucle ;
Z2 est absent ou une région de liaison d'acide nucléique ;
Z3 est une région d'ARN guide, dans lequel la région Z3 comprend une séquence d'acide nucléique qui peut guider la protéine Cas pour se lier spécifiquement à une molécule d'acide nucléique cible ;
Z5 est un acide nucléique simple brin clivable avec un marqueur détectable, dans lequel le marqueur détectable présente un état de détection différent lorsque l'acide nucléique simple brin clivable est clivé et non clivé, étant ainsi détecté ;
dans lequel, lorsque l'acide nucléique cible n'existe pas dans le système de détection, Z3 et Z5 forment une région de structure double brin appariée complémentaire, et lorsque l'acide nucléique cible existe dans le système de détection, Z3 et Z5 ne forment pas la région de structure double brin appariée complémentaire ;
Z4 est absent, ou une liaison chimique ou une région de liaison utilisée pour connecter Z3 et Z5 ;
« 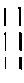 » est une liaison hydrogène pour l'appariement de bases complémentaires ;
et n est un entier positif avec n ≥ 1 ;
ii) un deuxième récipient et la protéine Cas située dans le deuxième récipient, qui est une protéine Cas avec une activité collatérale de clivage d'acide nucléique simple brin ;
iii) un troisième récipient facultatif et un tampon situé dans le troisième récipient.

13. Kit selon la revendication 12, dans lequel le kit comprend en outre :
iv) un quatrième récipient et la polymérase pour amplifier l'ADN cible située dans le quatrième récipient ;
v) un cinquième récipient facultatif et la transcriptase inverse pour la transcription inverse et/ou la transcriptase pour la transcription située dans le cinquième récipient ;
vi) un sixième récipient et les dNTP pour des réactions d'amplification et/ou des réactions de transcription inverse et/ou des NTP pour les réactions de transcription situés dans le sixième récipient.

14. Procédé de détection de molécules d'acide nucléique cibles dans un échantillon, qui comprend les étapes suivantes :
(i) fournir le système de détection pour détecter simultanément de multiples molécules d'acide nucléique cibles selon la revendication 1, et le système de détection contient en outre un échantillon à détecter ; et
(ii) détecter si la sonde de complexe d'acide nucléique rapporteur d'ARN guide dans le système de détection est clivée par la protéine Cas, et le clivage est un clivage collatéral (ou trans) pour l'acide nucléique simple brin ;
dans lequel si la sonde de complexe d'acide nucléique rapporteur d'ARN guide est clivée par la protéine Cas, cela signifie qu'il y a une molécule d'acide nucléique cible correspondante dans l'échantillon ; et si la sonde de complexe d'acide nucléique rapporteur d'ARN guide n'est pas clivée par la protéine Cas, cela signifie qu'il n'y a pas de molécule d'acide nucléique cible correspondante dans l'échantillon.

15. Procédé selon la revendication 14, dans lequel l'échantillon à analyser est un échantillon amplifié.
